(19) Europäisches Patentamt
European Patent Office
Office européen des brevets

(11) Publication number: **0 309 297 B1**

(12) # EUROPEAN PATENT SPECIFICATION

(45) Date of publication of patent specification: **09.11.94**

(51) Int. Cl.⁵: **C07K 7/00**, A61K 37/02, C07K 7/02

(21) Application number: **88308916.1**

(22) Date of filing: **26.09.88**

(54) **Therapeutic peptides.**

(30) Priority: **24.09.87 US 100571**

(43) Date of publication of application:
**29.03.89 Bulletin 89/13**

(45) Publication of the grant of the patent:
**09.11.94 Bulletin 94/45**

(84) Designated Contracting States:
**AT BE CH DE ES FR GB GR IT LI LU NL SE**

(56) References cited:
**US-A- 4 207 311**

**NATURE, vol. 316, 29th August 1985, pages 823-826, Mac Millan Journals,Basingstoke, GB; F. CUTTITTA et al.: "Bombesin-like peptides can function inhuman small-cell lung cancer"**

**AMERICAN JOURNAL OF PHYSIOLOGY, vol. 252, 1987, pages G439 - G442; P. HEINZ-ERIAN et al.: "[D-Phe12]bombesin analogues: a new class of bombesin receptorantagonists"**

(73) Proprietor: **The Administrators of The Tulane Educational Fund**
**1430 Tulane Avenue**
**New Orleans Louisiana 70112 (US)**

(72) Inventor: **Coy, David H.**
**4319 Perrier Street**
**New Orleans Louisiana 70115 (US)**
Inventor: **Moreau, Jacques-Pierre**
**159 Westboro Road**
**Upton Massachusetts 05168 (US)**

(74) Representative: **Deans, Michael John Percy et al**
**Lloyd Wise, Tregear & CO.**
**Norman House**
**105-109 Strand**
**London WC2R OAE (GB)**

EP 0 309 297 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**JOURNAL OF BIOLOGICAL CHEMISTRY, vol. 263, 1988, pages 5056-5060, Baltimore,US; D.H. COY et al.: "Probing peptide backbone function in bombesin"**

**Sasaki et al., J. Med. Chem. (1987), 30, 1162-1166.**

**Martiner et al., J. Med. Chem. (1985), 28, 1874-1879.**

## Description

This invention relates to novel peptides and their use e.g., in cancer therapy.

The amphibian peptide bombesin,

pGlu-Gln-Arg-Leu-Gly-Asn-Gln-Trp-Ala-Val-Gly-His-Leu-Met-$NH_2$

(Anastasi et al., Experientia 27:166-167 (1971)), is closely related to the mammalian gastrin-releasing peptides (GRP), e.g., the porcine GRP,

$H_2N$-Ala-Pro-Val-Ser-Val-Gly-Gly-Gly-Thr-Val-Leu-Ala-Lys-Met-Tyr-Pro-Arg-Gly-Asn-His-Trp-Ala-Val-Gly-His-Leu-Met-($NH_2$)

(McDonald et al., Biochem. Biophys. Res. Commun. 90:227-233 (1979)) and human GRP,

$H_2N$-Val-Pro-Leu-Pro-Ala-Gly-Gly-Gly-Thr-Val-Leu-Thr-Lys-Met-Tyr-Pro-Arg-Gly-Asn-His-Trp-Ala-Val-Gly-His-Leu-Met-$NH_2$.

Bombesin has been found to be an autocrine or paracrine mitotic factor for a number of human cancer cell lines, including small-cell lung carcinoma (SCLC) (Haveman et al., eds. Recent Results in Cancer Research - Peptide Hormones in Lung Cancer, Springer-Verlag, New York:1986). A number of these cancers are known to secrete peptide hormones related to GRP or bombesin. Consequently, antagonists to bombesin have been proposed as agents for the treatment of these cancers.

Cuttitta et al. demonstrated that a specific monoclonal antibody to bombesin inhibited in vivo the growth of a human small-cell lung cancer cell line xenografted to nude mice (Cuttitta et al., Cancer Survey 4:707-727 (1985)). In 3T3 murine fibroblasts which are responsive to the mitotic effect of bombesin, Zachary and Rozengurt observed that a substance P antagonist (Spantide) acted as a bombesin antagonist (Zachary et al., Proc. Natl. Acad. Sci. (USA), 82:7616-7620 (1985)). Heinz-Erian et al. replaced His at position 12 in bombesin with D-Phe and observed bombesin antagonist activity in dispersed acini from guinea pig pancreas (Heinz-Erian et al., Am. J. of Physiol. 252:G439-G442 (1987)). Rivier reported on work directed toward restricting the conformational freedom of the bioactive C-terminal decapeptide of bombesin by incorporating intramolecular disulfide bridges; however, Rivier mentioned that, so far, bombesin analogs with this modification fail to exhibit any antagonist activity (Rivier et al., "Competitive Antagonists of Peptide Hormones," in Abstracts of the International Symposium on Bombesin-Like Peptides in Health and Disease, Rome (October, 1987).

In the description hereinbelow, the following uncommon abbreviations are employed:

pGlu = 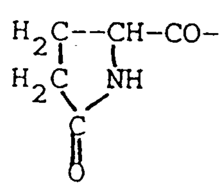 (pyroglutamate);

Nle = 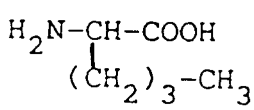 (norleucine)

Pal = 3-pyridyl-alanine

Nal = naphthylalanine

As will become clear from the description below, we have found that certain linear (i.e., non-cyclic) peptides which are analogs of a naturally occurring, biologically active bombesin having an active site and a

binding site responsible for the binding of bombesin to a receptor on a target cell, which analogs have a non-peptide bond instead of a peptide bond between an amino acid of the active site and an adjacent amino acid, are capable of binding to the receptor, so that the analog is capable of acting as a competitive inihibitor of naturally occurring bombesin by binding to the receptor and, by virtue of the non-peptide bond, failing to exhibit the in vivo activity of naturally occurring bombesin. (A detailed discussion of the chemistry of non-peptide bonds is given in Coy et al (1988) Tetrahedron 44,3:835-841, the disclosure of which is hereby effectively incorporated herein by reference).

Preferably, naturally occurring bombesin is characterized in that one or more amino acids in the amino terminal half of bombesin are hydrogen bonded to one or more amino acids in the carboxy terminal half of bombesin, and the non-peptide bond of the linear peptide decreases that hydrogen bonding, thereby destroying biological activity. It is believed that many of our linear peptides, details of which are given below are
analogs of bombesin whose biological activity depends at least in part on their ability to form tertiary "hairpin" configurations in which amino acids in the amino terminal ("left") half of the molecule are hydrogen bonded to amino acids in the carboxy terminal ("right") half of the molecule, and that the introduced pseudopeptide bond interferes with this hydrogen bonding, hindering the
formation of the hairpin configuration on which activity depends. One may expect the loss of the ability to hydrogen bond to affect the biological activity of the molecule either by the loss of structural stability conferred by the transannular bonding or by the inability of the backbone to hydrogen bond to the receptor. Additionally, the increased flexibility of the molecule about the reduced bond compared with the rigidity of the normal peptide amide bond is expected to alter the conformational integrity of the molecule and thus its biological activity.

The pseudopeptide bond can be introduced in a region involved in receptor binding, or in a non-binding region; it has been shown (Nagain et al., Peptides 8:1023-28 (1987)) that a pseudopeptide bond introduced in the binding region does not prevent binding. Generally, useful classes of peptides in which this modification can be made are those in which at least one amino acid involved in the active site is located in the carboxy terminal half of the molecule; the non-peptide bond is introduced between this amino acid and one adjacent to it.

According to a first aspect of this invention, there is provided a peptide having the amino acid formula:

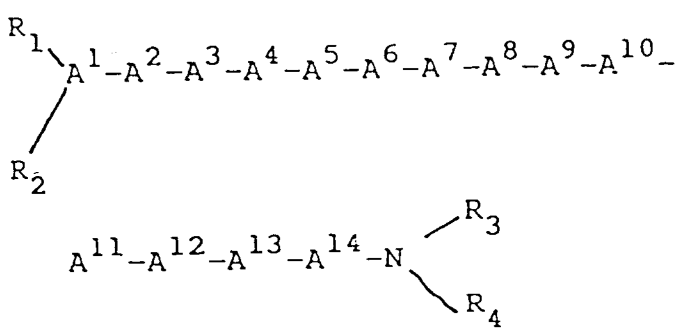

(I)

wherein,

- $A^1$ = pGlu, D or L or is deleted.
- $A^2$ = Gln, Asn, Gly, Ala, Leu, Ile, Nle, α-aminobutyric acid, Met, Val, Phe, p-X-Phe (X = F, Cl, Br, OH or $CH_3$), Trp, β-naphthylalanine or is deleted;
- $A^3$ = Arg, D-Arg, Lys, D-Lys or is deleted;
- $A^4$ = Gln, Asn, Gly, Ala, Leu, Ile, Nle, α-aminobutyric acid, Met, Val, Phe, p-X-Phe (X = F, Cl, Br, OH or $CH_3$), Trp, β-naphthylalanine or is deleted ;
- $A^5$ = Gln, Asn, Gly, Ala, Leu, Ile, Nle, α-aminobutyric acid, Met, Val, Phe, D-Phe, p-X-Phe (X = F, Cl, Br, OH or $CH_3$), Trp, β-naphthylalanine, D-Ala or is deleted;
- $A^6$ = Gln, Asn, Gly, Ala, D-Ala, N-Ac-D-Ala, Leu, Ile, Nle, α-aminobutyric acid, Met, Val, Phe, p-X-Phe (X = F, Cl, Br, OH or $CH_3$), Trp, p-Glu, β-naphthylalanine or is deleted;
- $A^7$ = Gln, Asn, Gly, Ala, Leu, Ile, Nle, α-aminobutyric acid, Met, Val, Phe, D-Phe, p-X-Phe (X = F, Cl, Br, OH or $CH_3$), Trp, Lys, His, or β-naphthylalanine;
- $A^8$ = Trp, Met;
- $A^9$ = Gln, Asn, Gly, Ala, Leu, Ile, Nle, α-aminobutyric acid, Met, Val, Phe, p-X-Phe (X = F, Cl, Br,

OH or CH ) Trp, or B-naphthylalanine, D or L;

$A^{10}$ = Gln, Asn, Gly, Ala, Leu, Ile, Nle, $\alpha$-aminobutyric acid, Met, Val, Phe, p-X-Phe (X = F, Cl, Br, OH or $CH_3$), Trp, Thr, or $\beta$-naphthylalanine;

$A^{11}$ = Gly, Phe, D or L;

$A^{12}$ = His, Phe, D or L, or p-X-Phe (X = F, Cl, Br, OH, $CH_3$), D or L;

$A^{13}$ = Gln, Asn, Gly, Ala, Leu, Ile, Nle, $\alpha$-aminobutyric acid, Met, Val, Phe, p-X-Phe (X = F, Cl, Br, OH or $CH_3$), Trp, or $\beta$-naphthylalanine;

$A^{14}$ = Gln, Asn, Gly, Ala, Leu, Ile, Nle, $\alpha$-aminobutyric acid, Met, Val, Phe, p-X-Phe (X = F, Cl, Br, OH or $CH_3$), Trp, or $\beta$-naphthylalanine;

provided that

each $R_1$, $R_2$, $R_3$, and $R_4$, independently, is H, $C_{1-12}$ alkyl, $C_{7-10}$ phenylalkyl, $COE_1$ (where $E_1$ is $C_{1-20}$ alkyl, $C_{3-20}$ alkenyl, $C_{3-20}$ alkinyl, phenyl, naphthyl, or $C_{7-10}$ phenylalkyl), or $COOE_2$ (where $E_2$ is $C_{1-10}$ alkyl or $C_{7-10}$ phenylalkyl), and $R_1$ and $R_2$ are bonded to the N-terminal amino acid of said peptide, which can be $A^1$, $A^2$, $A^3$, $A^4$, $A^5$, $A^6$, or $A^7$, and further provided that when one of $R_1$ or $R_2$ is $COE_1$ or $COOE_2$, the other must be H, and when one of $R_3$ or $R_4$ is $COE_1$ or $COOE_2$, the other must be H, and further provided that when $A^1$ = pGlu, $R_1$ must be H and $R_2$ must be the portion of Glu that forms the imine ring in pGlu; and for each of the residues $A^7$, $A^8$, $A^9$, $A^{11}$, $A^{12}$, and $A^{13}$, independently, the carbon atom participating in the amide bond between that residue and the nitrogen atom of the alpha amino group of the adjacent amino acid residue may be a carbonyl carbon or may be reduced to a methylene carbon, provided that at least one such carbon atom must be reduced to a methylene carbon; or a pharmaceutically acceptable salt thereof.

(Where no D- or L-isomeric designation is given herein, the naturally occurring L-isomer is intended.)

Preferably, the peptide has, for each of the residues $A^{11}$, $A^{12}$, and

$A^{13}$, independently, the carbon atom participating in the amide bond between that residue and the nitrogen atom of the alpha amino group of the adjacent amino acid residue which may be a carbonyl carbon or may be reduced to a methylene carbon, provided that at least one such carbon atom must be reduced to a methylene carbon; or a pharmaceutically acceptable salt thereof. Most preferably, the peptide has $A^1$ to $A^6$ inclusive deleted and the carbon atom participating in the amide bond between $Leu^{13}$ and $Leu^{14}$ is a methylene carbon; or is a pharmaceutically acceptable salt thereof.

In a second and alternative aspect of the present invention, we provide a peptide having the amino acid formula:

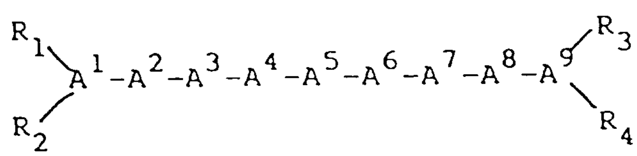

(II)

wherein $A^1$ is $A^6$ as defined in detail above, $A^2$ is $A^7$ as defined in detail above, $A^3$ is $A^8$ as defined in detail above, $A^4$ is $A^9$ as defined in detail above, $A^5$ is $A^{10}$ as defined in detail above, $A^6$ is $A^{11}$ as defined in detail above, $A^7$ is $A^{12}$ as defined in detail above, $A^8$ is $A^{13}$ as defined in detail above, and $A^9$ is $A^{14}$ as defined in detail above; provided that the carbon atom participating in the amide bond between the $A^8$ residue and the nitrogen atom of the alpha amino group of the adjacent amino acid residue is reduced to a methylene carbon; or a pharmaceutically acceptable salt thereof.

Peptides that contain a pseudopeptide bond substitution within the active site of the naturally occurring peptide are in general antagonists to the biological activity of the naturally occurring bombesin peptide. We have found, however, that the linear analog of bombesin BIM-26027 [$Val^{10}\psi Leu^{13}$[$CH_2NH$]$Leu^{14}$]BN is an agonist of the biological activity of naturally occurring bombesin.

In this description, the attached claims and in accompanying Table 1, the position of the non-peptide bond is indicated by the symbol $\psi$; $\psi$ is always shown preceding the amino acid which, in that peptide, is bonded by a non-peptide bond to the next sequential amino acid towards the C-terminus. The nature of the non-peptide bond (i.e. $CH_2NH$) is specified in between brackets at the site where the peptide bond replacement is taking place in the sequence. BN indicates bombesin.

According to a third alternative aspect of this invention, a peptide has the general formula I, wherein, for the residue $A^{10}$, the carbon atom participating in the amide bond between that residue and the nitrogen atom of the alpha amino group of the adjacent amino acid residue may be a carbonyl carbon or may be a non-peptide bond, provided that said non-peptide bond is said carbonyl carbon having been reduced to a

methylene carbon, further provided that at least one such carbon atom must be reduced to a methylene carbon; or a pharmaceutically acceptable salt thereof. Most preferred is the peptide having the formula

pGlu-Gln-Arg-Leu-Gly-Asn-Gln-Trp-Ala-Val-Gly-His-ψLeu[$CH_2NH$]Leu

i.e. [Val$^{10}$,ψLeu$^{13}$[$CH_2NH$]Leu$^{14}$]BN.

Other agonist analogs we have identified are peptides in which either the pseudopeptide bond is not located in the active site of the naturally occurring peptide, or in which two amino acid residues of the active site are replaced by statine or AHPPA.
(Statine has the chemical structure

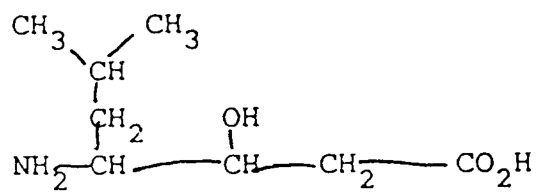

and statine-amide has the structure

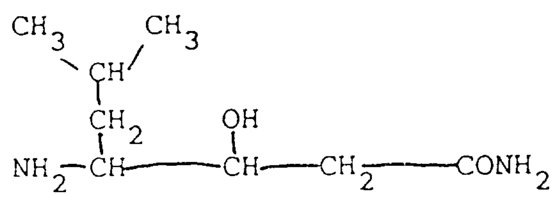

and AHPPA has the formula:
(3S,4S)-4-amino-3-hydroxy-5-phenylpentanoic acid.)

We provide, in a fourth alternative aspect of this invention, a peptide having the amino acid formula I, which is an analog of naturally occurring biologically active mammalian GRP or amphibian bombesin having an active site, said active site including the positions $A^9$,$A^{11}$, $A^{12}$,$A^{13}$, and $A^{14}$, and a binding site responsible for the binding of said bombesin to a receptor on a target cell, said analog having either (a) said non-peptide bond at residues other than within said active site, or (b) having at least one statine or AHPPA residue in place of two naturally occurring amino acids of said active site, and further provided that the peptide can contain statine or AHPPA when all bonds between amino acid residues are peptide bonds, and further provided that when an amino acid residue is statine or AHPPA, the amino acid to the right of it in the formula is deleted, so that said analog is capable of binding to said receptor and, by virtue of said statine or AHPPA residue, exhibiting enhanced in vivo biological activity compared to said naturally occurring bombesin.
Most preferred in this class is the peptide having the amino acid formula

pGlu-Gln-Arg-Leu-Gly-Asn-Gln-Trp-Ala-Val-Gly-His-[Sta$^{13}$,Des Met$^{14}$].

Bombesin antagonists and agonists in accordance with the aforesaid aspects of the invention are suitable for the treatment of all forms of cancer where bombesin-related substances act as autocrine or paracrine mitotic factors, especially pancreas and small-cell lung carcinoma.

In formula (1), when $R_1$, $R_2$, $R_3$ or $R_4$ is an aromatic, lipophilic group, the in vivo activity can be long lasting, and delivery of the compounds to the target tissue (e.g., the lungs) can be facilitated.

Other features and advantages of the invention will be apparent from the following description of the preferred embodiments.

We will first briefly describe the accompanying table.

The Table shows formulas for the pseuo-peptide analogues and results of in vitro inhibition of [$^{125}$I] GRP binding to cerebral cortex and 3T3 fibroblast or murine fibroblast bombesin receptors, and bombesin-stimulated [$^{3}$H]Thymidine uptake by cultured 3T3 cells.

We now describe the structure, synthesis, and use of the preferred embodiments of the invention.

The peptides of the invention all have a non-peptide bond in at least one of the indicated positions, except for the statine or AHPPA substituted analogs, such as [sta$^{13}$-des Met$^{14}$] bombesin. By non-peptide bond is meant that the carbon atom participating in the bond between two residues is reduced from a carbonyl carbon to a methylene carbon. The peptide bond reduction method which yields this non-peptide bond is described in Coy et al., U.S. patent application, Serial No. 879,348, assigned to the present applicants, hereby incorporated by reference. Any one or all of the amino acids in positions 1 to 6 inclusive may be deleted from the peptides.

The peptides of the invention can be provided in the form of pharmaceutically acceptable salts. Examples of preferred salts are those with therapeutically acceptable organic acids, e.g., acetic, lactic, maleic, citric, malic, ascorbic, succinic, benzoic, salicylic, methanesulfonic, toluenesulfonic, or pamoic acid, as well as polymeric acids such as tannic acid or carboxymethyl cellulose, and salts with inorganic acids such as the hydrohalic acids, e.g., hydrochloric acid, sulfuric acid, or phosphoric acid.

The synthesis of the bombesin antagonist

pGlu-Gln-Arg-Leu-Gly-Asn-Gln-Trp-Ala-Val-Gly-His-
ψLeu[$CH_2$-NH]Leu-$NH_2$

follows. Other bombesin antagonists and agonists can be prepared by making appropriate modifications of the following synthetic method.

The first step is the preparation of the intermediate

pGlu-Gln-Arg(tosyl)-Leu-Gly-Asn-Gln-
Trp-Ala-Val-Gly-His(benzyloxycarbonyl)-ψLeu[$CH_2$NH]
Leu-benzhydrylamine resin,

as follows.

Benzhydrylamine-polystyrene resin (Vega Biochemicals, Inc.) (0.97 g, 0.5 mmole) in the chloride ion form is placed in the reaction vessel of a Beckman 990B peptide synthesizer programmed to perform the following reaction cycle: (a) methylene chloride; (b) 33% trifluoroacetic acid (TFA) in methylene chloride (2 times for 1 and 25 min. each); (c) methylene chloride; (d) ethanol; (e) methylene chloride; and (f) 10% triethylamine in chloroform.

The neutralized resin is stirred with alpha-t-butoxycarbonyl(Boc)-leucine and diisopropylcarbodiimide (1.5 mmole each) in methylene chloride for 1 hour, and the resulting amino acid resin is then cycled through steps (a) to (f) in the above wash program. Boc-leucine aldehyde (1.25 mmoles), prepared by the method of Fehrentz and Castro, Synthesis, p. 676 (1983), is dissolved in 5 ml of dry dimethylformamide (DMF) and added to the resin TFA salt suspension followed by the addition of 100 mg (2 mmoles) of sodium cyanoborohydride (Sasaki and Coy, Peptides 8:119-121 (1987); Coy et al., id.). After stirring for 1 hour, the resin mixture is found to be negative to ninhydrin reaction (1 min.), indicating complete derivatization of the free amino group.

The following amino acids (1.5 mmole) are then coupled successively in the presence of diisopropylcarbodiimide (1.5 mmole), and the resulting amino acid resin is cycled through washing/deblocking steps (a) to (f) in the same procedure as above: Boc-His(benzyloxycarbonyl), Boc-Gly, Boc-Val, Boc-Ala, Boc-Trp, Boc-Gln (coupled in the presence of equivalent of hydroxybenzotriazole), Boc-Asn (coupled in the presence of 1 equivalent of hydroxybenzotriazole), Boc-Gly (coupled as a 6 M excess of the p-nitrophenyl ester), Boc-Leu, Boc-Arg(tosyl), Boc-Gln (coupled as a 6 M excess of the p-nitrophenylester), and pGlu. The completed resin is then washed with methanol and air dried.

The resin described above (1.6 g, 0.5 mmole) is mixed with anisole (5 ml) and anhydrous hydrogen fluoride (35 ml) at 0 °C and stirred for 45 min. Excess hydrogen fluoride is evaporated rapidly under a stream of dry nitrogen, and free peptide is precipitated and washed with ether. The crude peptide is

dissolved in a minimum volume of 2 M acetic acid and eluted on a column (2.5 x 100 mm) of Sephadex G-25 (Pharmacia Fine Chemicals, Inc.). Fractions containing a major component by uv absorption and thin layer chromatography (TLC) are then pooled, evaporated to a small volume and applied to a column (2.5 x 50 cm) of octadecylsilane-silica (Whatman LRP-1, 15-20 μm mesh size).

The peptide is eluted with a linear gradient of 0-30% acetonitrile in 0.1% trifluoroacetic acid in water. Fractions are examined by TLC and analytical high performance liquid chromatography (HPLC) and pooled to give maximum purity. Repeated lyophilization of the solution from water gives 60 mg of the product as a white, fluffy powder.

The product is found to be homogeneous by HPLC and TLC. Amino acid analysis of an acid hydrolysate confirms the composition of the peptide. The presence of the ψLeu[$CH_2$NH]Leu bond is demonstrated by fast atom bombardment mass spectrometry.

pGlu-Gln-Arg-Leu-Gly-Asn-Gln-Trp-ψAla[$CH_2$NH]Val-Gly-His-Leu-Met-$NH_2$ and pGlu-Gln-Arg-Leu-Gly-Asn-Gln-Trp-Ala-Val-Gly-His-ψLeu[$CH_2$NH]Met-$NH_2$

are prepared in similar yields in an analogous fashion by appropriately modifying the above procedure.

A statine or AHPPA residue can be substituted in place of any two amino acids of the peptide, where the peptide contains no pseudopeptide bonds. For example, [sta$^{13}$-des Met$^{14}$] bombesin was prepared in an analagous fashion by first coupling statine to the resin and then proceeding with the addition of Boc-His-(benzylocarbonyl). Statine or Boc-statine can be synthesized according to the method of Rich et al., 1978, J. Organic Chem. 43; 3624; and Rich et al., 1980, J. Med. Chem. 23: 27, and AHPPA can be synthesized according to the method of Hui et al., 1987, J. Med. Chem. 30: 1287.

Solid-phase synthesis of the peptide pGlu-Gln-Arg-Leu-Gly-Asn-Gln-Trp-Ala-Val-Gly-His-Sta-$NH_2$ was accomplished through the use of the following procedures in which alpha-t-butoxycarbonyl statine (prepared by the procedure of Rich et al., J. Org. Chem. 1978, 43, 3624) is first coupled to methylbenzhydrylamine-polystyrene resin. After acetylation, the intermediate

p-Glu-Gln-Arg(tosyl)-Leu-Gly-Asn-Gln-Trp-Ala-Val-Gly-His(benzyloxycarbonyl)-Sta-methylbenzhydrylamine resin

is prepared. The synthetic procedure used for this preparation follows in detail:

1. Incorporation of alpha-t-butoxycarbonyl statine on methylbenzhydrylamine resin.

Methylbenzhydrylamine-polystyrene resin (Vega Biochemicals, Inc.) (1.0 g, 0.73 mmol) in the chloride ion form is placed in the reaction vessel of a Vega 250C Coupler peptide synthesizer. The synthesizer was programmed to perform the following reactions: (a) methylene chloride; (b) 10% triethylamine in chloroform; (c) methylene chloride; and (d) dimethylformamide.

The neutralized resin is mixed for 18 hours with the preformed active ester made from alpha-t-butoxycarbonyl statine (1.46 mmol), diisopropyl carbodiimide (2 mmol), and hydroxybenzotriazole hydrate (1.46 mmol in dimethylformamide at 0° C. for one hour. The resulting amino acid resin is washed on the synthesizer with dimethylformamide and then methylene chloride. The resin mixture at this point was found by the Kaiser ninhydrin test (5 minutes) to have an 84% level of statine incorporation on the resin.

Acetylation was performed by mixing the amino-acid resin for 15 minutes with N-acetyl imidazole (5 mmol) in methylene chloride. Derivitization to the 94-99% level of the free amino groups of the resin was indicated by the Kaiser ninhydrin test (5 minutes). The Boc-statine-resin is then washed with methylene chloride.

2. Couplings of the Remaining Amino Acids.

The peptide synthesizer is programmed to perform the following reaction cycle: (a) methylene chloride; (b) 33% trifluroacetic acid (TFA) in methylene chloride (2 times for 5 and 25 min. each); (c) methylene chloride; (d) isopropyl alcohol; (e) 10% triethylamine in chloroform; and (f) methylene chloride.

The following amino acids (2.19 mmol) are then coupled successively by diisopropyl carbodiimide (4 mmol) alone or diisopropyl carbodiimide (4 mmol) plus hydroxybenzotriazole hydrate (1.47 or 0.73 mmol) and the resulting peptide-resin is washed on the synthesizer with dimethylformamide and then methylene chloride, and then cycled through the washing and deblocking steps (a) to (f) in the procedure described above.

Boc-His (benzyloxycarbonyl) (coupled in the presence of 2 equivalents hydroxybenzotriazole); Boc-Gly; Boc-Val; Boc-Ala; Boc-Trp; Boc-Gln and Boc-Asn (coupled as the preformed hydroxybenzotriazole active esters made by reaction at 0° C. for one hour with 1 equivalent hydroxybenzotriazole hydrate); Boc-Gly; Boc-Leu; Boc-Arg(tosyl), Boc-Gln, and pGlu (also coupled as the preformed active esters of hydroxybenzotriazole made by reaction at 0° C. for one hour with 1 equivalent hydroxybenzotriazole hydrate). The completed peptide-resin is then washed with methanol and air dried.

The peptide-resin described above (1.60 g, 0.73 mmol) is mixed with anisole (2.5 mL), dithioerythritol (50 mg), and anhydrous hydrogen fluoride (30 mL) at 0° C. for one hour. Excess hydrogen fluoride is evaporated rapidly under a stream of dry nitrogen, and the free peptide is precipitated and washed with ether. The crude peptide is dissolved in 100 mL of 1 M acetic acid and the solution is then evaporated under reduced pressure. The crude peptide is dissolved in a minimum volume of methanol/water 1/1 and triturated with 10 volumes of ethyl acetate.

The triturated peptide is applied to a column (9.4 mm I.D. x 50 cm) of octadecylsilane-silica (Whatman Partisil 10 ODS-2 M 9). The peptide is eluted with a linear gradient of 20-80% of 20/80 0.1% trifluoroacetic acid/acetonitrile in 0.1% trifluoroacetic acid in water. Fractions are examined by TLC and analytical high performance liquid chromatography (HPLC) and pooled to give maximum purity. Lyophilization of the solution from water gives 77 mg of the product as a white fluffy powder.

Other compounds can be prepared as above and tested for effectiveness as agonists or antagonists in the following test program.

Phase 1 - 3T3 Peptide Stimulated [$^3$H] Thymidine Uptake Assay

Cell Culture. Stock cultures of Swiss 3T3 cells (American Type culture Collection No. CCL 92) are grown in Dulbecco's Modified Eagles Medium (DMEM) supplemented with 10% fetal calf serum in humidified atmosphere of 10% $CO_2$/90% air at 37°C. For experimental use, the cells are seeded into 24-well cluster trays and used four days after the last change of medium. The cells are arrested in the G1/G0 phase of the cell cycle by changing to serum-free DMEM 24 hours prior to the thymidine uptake assay.

Assay of DNA Synthesis. The cells are washed twice with 1ml aliquots of DMEM (-serum) then incubated with DMEM (-serum), 0.5$\mu$M [methyl-$^3$H] thymidine (20Ci/mmole, New England Nuclear), bombesin (1nM), and four concentrations of the test compounds (1, 10, 100, 1000nM) in a final volume of 0.5ml. After 28 hours at 37°C, [methyl-$^3$H] thymidine incorporation into acid-insoluble pools is assayed as follows. The cells are washed twice with ice-cold 0.9% NaCl (1ml aliquots), and acid soluble radioactivity is removed by a 30 min. (4°C) incubation with 5% trichloroacetic acid (TCA). The cultures are then washed once (1ml) with 95% ethanol and solubilized by a 30 min. incubation (1ml) with 0.1N NaOH. The solubilized material is transferred to vials containing 15ml ScintA (Packard), and the radioactivity is determined by liquid scintillation spectrometry.

Phase 2 - Small Cell Carcinoma (SCLC) - Bombesin Stimulated [$^3$H] Thymidine Uptake Assay

Cell Culture. Cultures of the human cell carcinoma cell line (NCI-H69) (obtained from the American Type Culture Association) are maintained in RPMI 1640 medium supplemented with 10% fetal calf serum in 10% $CO_2$/90% air at 37°C. Twenty-four hours prior to assay, the cells are washed with serum-free medium and seeded in 24-well cluster trays.

Assay of DNA Synthesis. Bombesin (1nM), 0.5$\mu$M [methyl-$^3$H] thymidine (20 Ci/mmole, New England Nuclear), and four concentrations of the test compounds (1, 10, 100, 1000nM) are added to the cultures to achieve a final volume of 0.5 ml. After a 28 hr incubation at 37°C, the cells are collected onto GF/B glass fiber filters, and the DNA is precipitated with ice-cold TCA. [$^3$H] thymidine incorporation into acid-insoluble fractions of DNA is determined by liquid scintillation spectrometry.

Phase 3 - Peptide-Induced Pancreatitis

Male, Sprague-Dawley rats (250g) are used for these experiments. The test compound, or 0.9% NaCl is administered s.c. 15 min. prior to the bombesin injection. Bombesin injections are given s.c. at a dose of 10

µg/kg, and blood samples are obtained at 1 hr.30 min., 3hr. and 6hr. Plasma amylase concentration are determined by the Pantrak Amylase test.

Phase 4- In Vitro Inhibition of [$^{125}$I] Gastrin Releasing Peptide (GRP) Binding to Bombesin Receptors

Membranes from various tissues (rat brain, rat pancreas, rat anterior pituitary, SCLC, 3T3 cells) are prepared by homogenization in 50mM TrisHCl containing 0.1% bovine serum albumin and 0.1mg/ml bacitracin followed by two centrifugations (39,000xgx15 min., 4°C) with an intermediate resuspension in fresh buffer. For assay, aliquots (0.8ml) are incubated with 0.5nM [$^{125}$I]GRP ('2000 Ci/mmol, Amersham Corp.) and various concentrations of the test compounds in a final volume of 0.5ml. After a 30 minute incubation at 4°C, the binding reaction is terminated by rapid filtration through Whatman GF/C filters that have been pre-soaked in 0.3% aqueous polethyleneimine to reduce the level of nonspecific binding. The filters and tubes are washed three times with 4ml aliquots of ice-cold buffer, and the radioactivity trapped on the filters is counted by gamma-spectrometry. Specific binding is defined as the total [$^{125}$I]GRP bound minus that bound in the presence of 1000nM bombesin.

Phase 5- Inhibition of Gastrin Release

The stomachs of anesthetized rats are perfused with saline collected over 15 minute periods via pyloric cannulation while the test peptide is infused through the femoral vein for periods between 0 and 150 minutes.

Results of Tests of Test Peptides

A number of analogs of bombesin were synthesized and tested in one or more of the above-described Phase 1-5 assays; the results of Phase 1, 2 and 4 tests are given in Table 1 attached hereto, Brain and 3T3 GRP receptor and thymidine uptake data are expressed in IC50 (nM). In Table 1, EC50 stands for the concentration at which 50% of the efficacy is reached; while IC50 stands for the concentration required for 50% inhibition.

In Table 1, it can be seen that a preferred placement of the non-peptide bond in bombesin analogs is at the 13-14 position; two of the most active analogs (as indicated by a low GRP receptor IC50 value) are BIM-26027 and BIM-26028. However, BIM-26027 causes proliferation of cancer cells (see Table 1, under thymidine uptake), and therefore is an agonist and not an antagonist. In general, compounds having the non-peptide bond at any position other than the active site of the peptide are agonists rather than antagonists. Table I also shows that when statine replaces the $A^{13}$ and $A^{14}$ residues of bombesin, the resultant bombesin analog BIM-26096 causes proliferation of cancer cells and is therefore an agonist. Bombesin superagonists may be useful in cancer therapy, as suggested by Alexander et al., 1988, Cancer Research 48: 1439-1441, and Alexander et al., 1988, Pancreas 3:297-302, hereby incorporated by reference. Alexander et al. show that chronic bombesin treatment inhibited the growth of human ductal adenocarcinoma transplanted into athymic mice. These results were surprising for bombesin stimulates growth of normal pancreas tissue. The demonstration of both stimulatory and inhibitory activity suggests that bombesin interacts differently in normal and neoplastic pancreatic tissues.

These observations prompted us to evaluate the affect of BIM-26096, a bombesin analogue which has bombesin-like agonist activity, on the in vitro growth of a pancreatic tumor cell line (AR42J, A.T.C.C. No. CRL1492. For these experiments, AR42J cells were subcultured into a 24-well culture plate in Dulbecco's modified Eagle's medium containing 10% fetal calf serum containing various concentrations (0.1-100nM) of BIM-26096. After a 36 hr incubation the cells were removed with a trypsin/EDTA solution and the number of cells were determined using a Coulter Counter. The results are shown below:

| Treatment | Cell Count (% Control) |
|---|---|
| control | 100 |
| BIM-26096 (0.1 nM) | 78 |
| BIM-26096 (1.0 nM) | 73 |
| BIM-26096 (10 nM) | 56 |
| BIM-26096 (100 nM) | 52 |

These results indicate that the bombesin agonist, BIM-26096, has in vitro antiproliferative activity against the AR42J rat pancreas tumor.

Finally, Table 1 also shows that bond placement, while important, is not the only factor influencing activity, and that amino acid substitutions at some positions exert influence as well; this is illustrated by BIM-26030, with Gly in position 11, which exhibited no antagonist activity. Thus the non-peptide bond placement guidelines given herein should be used in conjunction with the routine assays described above to select useful antagonists or agonists.

In a phase 5 assay, above, the results of which are not given in Table 1, BIM-26028 was shown to be a potent inhibitor of bombesin - stimulated gastric acid secretion.

The disclosed peptides may be administered to a mammal, particularly a human, in one of the traditional modes (e.g., orally, parenterally, transdermally, or transmucosally), in a sustained release formulation using a biodegradable biocompatible polymer, or by on-site delivery (e.g., in the case of anti-cancer bombesin to the lungs) using micelles, gels and liposomes.

The bombesin antagonists and agonists of the invention are suitable for the treatment of all forms of cancer where bombesin-related substances act as autocrine or paracrine mitotic agents, particularly small-cell lung carcinoma. The peptides can also be used for the inhibition of gastric acid secretion, the symptomatic relief and/or treatment of exocrine pancreatic adenocarcinoma, and the restoration of appetite to cachexic patients. The peptides can be administered to a human patient in a dosage of 0.5 µg/kg/day to 5 mg/kg/day. For some forms of cancer, e.g., small cell lung carcinoma, the preferred dosage for curative treatment is 250mg/patient/day.

## TABLE 1

| Code | Structure | Brain GRP Receptor IC50(nM) | 3T3 GRP Receptor IC50(nM) | Thym. Uptake IC50(nM) |
|---|---|---|---|---|
| BIM-26025: | [ψ$His^{12}$[$CH_2NH$]$Leu^{13}$, $Leu^{14}$]BN (1-14) | >1000 | | |
| BIM-26026: | [ψ$Ala^{9}$[$CH_2NH$]$Val^{10}$, $Leu^{14}$]BN(1-14) | >1000 | | 1574 |
| BIM-26028: | [ψ$Leu^{13}$[$CH_2NH$]$Leu^{14}$]BN(1-14) | 13 | | 14.7 |
| BIM-26030: | [ψ$Gly^{11}$[$CH_2NH$]$His^{12}$, $Leu^{14}$]BN(1-14) | >1000 | | |
| BIM-26034: | [ψ$Gln^{7}$[$CH_2NH$]$Trp^{8}$]BN(1-14) | >1000 | | |
| BIM-26036: | [Des-$pGlu^{1}$, $Gln^{2}$, ψ$Ala^{9}$[$CH_2NH$]$Val^{10}$, $Phe^{12}$]BN(1-14) | >1000 | | |
| BIM-26046: | [ψ$Gly^{11}$[$CH_2NH$]D-$Phe^{12}$, $Leu^{14}$]BN(1-14) | >1000 | | |
| BIM-26048: | [ψD-$Phe^{12}$[$CH_2NH$]$Leu^{13}$, $Leu^{14}$]BN(1-14) | >1000 | | |
| BIM-26061: | [D-$pGlu^{1}$, D-$Ala^{5}$, ψ$Leu^{7}$[$CH_2NH$]$Met^{8}$]BN(1-14) | >1000 | | |
| BIM-26062: | [ψ$Phe^{13}$[$CH_2NH$]$Leu^{14}$]BN(1-14) | >1000 | | 437 |
| BIM-26063: | = BIM-26028 | | >1000 | |

| | | | | |
|---|---|---|---|---|
| BIM-26068: | [ψLeu$^{13}$[$CH_2NH$]Phe$^{14}$]BN(1-14) | 2.9 | | 70 |
| BIM-26075: | [D-Phe$^{11}$, ψLeu$^{13}$[$CH_2NH$]Leu$^{14}$]BN(1-14) | >1000 | | |
| BIM-26076: | [Phe$^{11}$, ψLeu$^{13}$[$CH_2NH$]Leu$^{14}$]BN(1-14) | >1000 | | |
| BIM-26077: | [D-Ala$^{5}$, ψLeu$^{13}$[$CH_2NH$]Leu$^{14}$]BN(1-14) | 517 | 196 | 1001 |
| BIM-26086: | [D-Phe$^{12}$, ψLeu$^{13}$[$CH_2NH$]Leu$^{14}$]BN(1-14) | >1000 | | |
| BIM-26092: | [His$^{7}$, ψLeu$^{13}$[$CH_2NH$]Met$^{14}$]BN(5-14) | | 242 | 466 |
| BIM-26093: | [D-Ala$^{5}$, His$^{7}$, ψLeu$^{13}$[$CH_2NH$]Leu$^{14}$]BN(5-14) | | 82 | 171 |
| BIM-26096: | [Sta$^{13}$, DesMet$^{14}$]BN(1-14) | 33 | | agonist EC50=3nM |
| BIM-26098: | [Lys$^{7}$, ψLeu$^{13}$[$CH_2NH$]Met$^{14}$]BN(7-14) | 1000 | | |
| BIM-26099: | [ψLeu$^{13}$[$CH_2NH$]Met$^{14}$]BN(1-14) | | 73 | 78 |
| BIM-26100: | [P-Glu$^{6}$, ψPhe$^{13}$[$CH_2NH$]Leu$^{14}$]BN(6-14) | | 74 | 22 |
| BIM:26101: | [P-Glu$^{6}$, ψLeu$^{13}$[$CH_2NH$]Leu$^{14}$]BN(6-14) | | 17.9 | 257 |
| BIM-26102: | [Leu$^{7}$, Thr$^{10}$, ψPhe$^{13}$[$CH_2NH$]Met$^{14}$]BN(5-14) | | 184 | >1000 |

## Claims

**1.** A peptide having the amino acid formula:

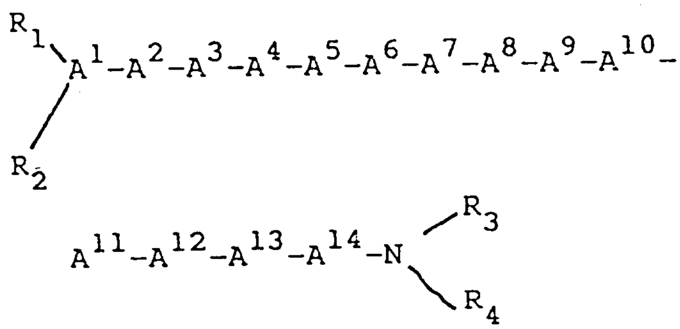

(I)

wherein,

$A^1$ = pGlu, D or L or is deleted.

$A^2$ = Gln, Asn, Gly, Ala, Leu, Ile, Nle, $\alpha$-aminobutyric acid, Met, Val, Phe, p-X-Phe (X = F, Cl, Br, OH or $CH_3$), Trp, $\beta$-naphthylalanine or is deleted;

$A^3$ = Arg, D-Arg, Lys, D-Lys or is deleted;

$A^4$ = Gln, Asn, Gly, Ala, Leu, Ile, Nle, $\alpha$-aminobutyric acid, Met, Val, Phe, p-X-Phe (X = F, Cl, Br, OH or $CH_3$), Trp, $\beta$-naphthylalanine or is deleted ;

$A^5$ = Gln, Asn, Gly, Ala, Leu, Ile, Nle, $\alpha$-aminobutyric acid, Met, Val, Phe, D-Phe, p-X-Phe (X = F, Cl, Br, OH or $CH_3$), Trp, $\beta$-naphthylalanine, D-Ala or is deleted;

$A^6$ = Gln, Asn, Gly, Ala, D-Ala, N-Ac-D-Ala, Leu, Ile, Nle, $\alpha$-aminobutyric acid, Met, Val, Phe, p-X-Phe (X = F, Cl, Br, OH or $CH_3$), Trp, p-Glu, $\beta$-naphthylalanine or is deleted;

$A^7$ = Gln, Asn, Gly, Ala, Leu, Ile, Nle, $\alpha$-aminobutyric acid, Met, Val, Phe, D-Phe, p-X-Phe (X = F, Cl, Br, OH or $CH_3$), Trp, Lys, His, or $\beta$-naphthylalanine;

$A^8$ = Trp, Met;

$A^9$ = Gln, Asn, Gly, Ala, Leu, Ile, Nle, $\alpha$-aminobutyric acid, Met, Val, Phe, p-X-Phe (X = F, Cl, Br, OH or CH ) Trp, or B-naphthylalanine, D or L;

$A^{10}$ = Gln, Asn, Gly, Ala, Leu, Ile, Nle, $\alpha$-aminobutyric acid, Met, Val, Phe, p-X-Phe (X = F, Cl, Br, OH or $CH_3$), Trp, Thr, or $\beta$-naphthylalanine;

$A^{11}$ = Gly, Phe, D or L;

$A^{12}$ = His, Phe, D or L, or p-X-Phe (X = F, Cl, Br, OH, $CH_3$), D or L;

$A^{13}$ = Gln, Asn, Gly, Ala, Leu, Ile, Nle, $\alpha$-aminobutyric acid, Met, Val, Phe, p-X-Phe (X = F, Cl, Br, OH or $CH_3$), Trp, or $\beta$-naphthylalanine;

$A^{14}$ = Gln, Asn, Gly, Ala, Leu, Ile, Nle, $\alpha$-aminobutyric acid, Met, Val, Phe, p-X-Phe (X = F, Cl, Br, OH or $CH_3$), Trp, or $\beta$-naphthylalanine;

provided that

each $R_1$, $R_2$, $R_3$, and $R_4$, independently, is H, $C_{1-12}$ alkyl, $C_{7-10}$ phenylalkyl, $COE_1$ (where $E_1$ is $C_{1-20}$ alkyl, $C_{3-20}$ alkenyl, $C_{3-20}$ alkinyl, phenyl, naphthyl or $C_{7-10}$ phenylalkyl), or $COOE_2$ (where $E_2$ is $C_{1-10}$ alkyl or $C_{7-10}$ phenylalkyl), and $R_1$ and $R_2$ are bonded to the N-terminal amino acid of said peptide which can be $A^1$, $A^2$, $A^3$, $A^4$, $A^5$, $A^6$, or $A^7$, and further provided that when one of $R_1$ or $R_2$ is $COE_1$ or $COOE_2$, the other must be H, and when one of $R_3$ or $R_4$ is $COE_1$ or $COOE_2$, the other must be H, and further provided that when $A^1$ = pGlu, $R_1$ must be H and $R_2$ must be the portion of Glu that forms the imine ring in pGlu; and for each of the residues $A^7$, $A^8$, $A^9$, $A^{11}$, $A^{12}$, and $A^{13}$, independently, the carbon atom participating in the amide bond between that residue and the nitrogen atom of the alpha amino group of the adjacent amino acid residue may be a carbonyl carbon or may be reduced to a methylene carbon, provided that at least one such carbon atom must be reduced to a methylene carbon; or a pharmaceutically acceptable salt thereof.

**2.** A peptide according to Claim 1, wherein $A^1$ to $A^6$ inclusive are deleted and the carbon atom participating in the amide bond between $Leu^{13}$ and $Leu^{14}$ is a methylene carbon; or a pharmaceutically acceptable salt thereof.

**3.** A peptide according to Claim 1, wherein, for each of said residues $A^{11}$, $A^{12}$, and $A^{13}$, independently, the carbon atom
participating in the amide bond between that residue and the nitrogen atom of the alpha amino group of the adjacent amino acid residue may be a carbonyl carbon or may be reduced to a methylene carbon, provided that at least one such carbon atom must be reduced to a methylene carbon; or a pharmaceutically acceptable salt thereof.

**4.** A peptide having the amino acid formula:

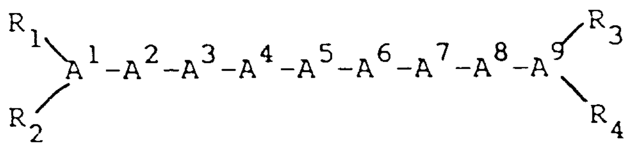

(II)

wherein $A^1$ is $A^6$ of Claim 1, $A^2$ is $A^7$ of Claim 1, $A^3$ is $A^8$ of Claim 1, $A^4$ is $A^9$ of Claim 1, $A^5$ is $A^{10}$ of Claim 1, $A^6$ is $A^{11}$ of Claim 1, $A^7$ is $A^{12}$ of Claim 1, $A^8$ is $A^{13}$ of Claim 1, and $A^9$ is $A^{14}$ of Claim 1; provided that the carbon atom participating in the amide bond between the $A^8$ residue and the nitrogen atom of the alpha amino group of the adjacent amino acid residue is reduced to a methylene carbon; or a pharmaceutically acceptable salt thereof.

**5.** A peptide according to Claim 1 wherein, for the residue $A^{10}$, the carbon atom participating in the amide bond between that residue and the nitrogen atom of the alpha amino group of the adjacent amino acid residue may be a carbonyl carbon or may be a non-peptide bond, provided that said non-peptide bond is said carbonyl carbon having been reduced to a methylene carbon, further provided that at least one such carbon atom must be reduced to a methylene carbon; or a pharmaceutically acceptable salt thereof.

**6.** A peptide having the amino acid formula

pGlu-Gln-Arg-Leu-Gly-Asn-Gln-Trp-Ala-Val-Gly-His-
ψLeu[$CH_2$NH]Leu.

**7.** A peptide having the amino acid formula of Claim 1 which is an analog of naturally occurring, biologicallly active mammalian GRP or amphibian bombesin having an active site, said active site including the positions $A^9$,$A^{11}$, $A^{12}$,$A^{13}$, and $A^{14}$, and a binding site responsible for the binding of said bombesin to a receptor on a target cell, said analog having either (a) said non-peptide bond at residues other than within said active site, or (b) having at least one statine or AHPPA residue in place of two naturally occurring amino acids of said active site, and further provided that the peptide can contain statine or AHPPA when all bonds between amino acid residues are peptide bonds, and further provided that when an amino acid residue is statine or AHPPA, the amino acid to the right of it in the formula is deleted, so that said analog is capable of binding to said receptor and, by virtue of said statine or AHPPA residue, exhibiting enhanced in vivo biological activity compared to said naturally occurring bombesin.

**8.** A peptide having the amino acid formula

pGlu-Gln-Arg-Leu-Gly-Asn-Gln-Trp-Ala-Val-Gly-His-
Statine[$Sta^{13}$,Des $Met^{14}$]BN.

9. A method of synthesizing a peptide having the amino acid formula defined in Claim 1; said method comprising the steps of (a) preparing benzyhydrylamine-polystyrene resin, (b) neutralizing said resin and coupling a chosen amino acid for said $A^{14}$ position to said resin, (c) coupling a chosen amino acid for said $A^{13}$ position to said $A^{14}$-resin, (d) derivatizing the free amino group of said $A^{13}$-$A^{14}$-resin, and (e) coupling successively to said derivatized amino acid resin a chosen amino acid for each of said $A^{12}$ to $A^{1}$ positions by repeating steps (d) and (e) for each of said amino acid positions, the carbon atom participating in the amide bond between any one of residues $A^{7}$, $A^{8}$, $A^{9}$, $A^{11}$, $A^{12}$ and $A^{13}$ and the nitrogen atom of the alpha amino group of the adjacent amino acid residue being reduced to a methylene carbon.

10. The method of Claim 9, wherein any or all of said positions $A^{6}$ to $A^{1}$ inclusive are deleted.

11. A method of synthesizing a peptide having the amino acid formula of Claim 1 which is an analog of naturally occurring, biologically active mammalian GRP or amphibian bombesin, which has an active site including the positions $A^{9}$, $A^{11}$, $A^{12}$, $A^{13}$, and $A^{14}$, and a binding site responsible for the binding to a receptor on a target cell, said method comprising either the steps of (a) preparing benzyhydrylamine-polystyrene resin, (b) neutralizing said resin and coupling a chosen amino acid for said $A^{14}$ amino acid position or alpha-t-butoxycarbonyl statine to said resin, (c) coupling a chosen amino acid for said $A^{13}$ position or said statine for said $A^{13}$ position to said $A^{14}$-resin, (d) derivatizing the free amino group of said $A^{13}$-$A^{14}$-resin or said statine-resin, and (e) coupling successively to said derivatized amino acid or statine resin a chosen amino acid for each of said $A^{12}$ to $A^{1}$ inclusive positions by repeating steps (d) and (e) for each of said amino acid positions; provided that when said statine is coupled to said resin, said statine-resin is acetylated before derivatization to free amino of groups; and provided that at least one statine or AHPPA residue replaces two naturally occuring amino acids of said active site; or the steps of the method of Claim 9 when the non-peptide bond is outside of said active site.

12. A peptide according to any of Claims 1 to 8 for use in the treatment of cancer in patients.

13. Use of a peptide according to any of Claims 1 to 8 for the manufacture of a medicament for use in the treatment of cancer in patients.

14. A pharmaceutical composition comprising a peptide according to any of Claims 1 to 8, together with a pharmaceutically acceptable carrier.

## Patentansprüche

1. Peptid der Aminosaüreformel :

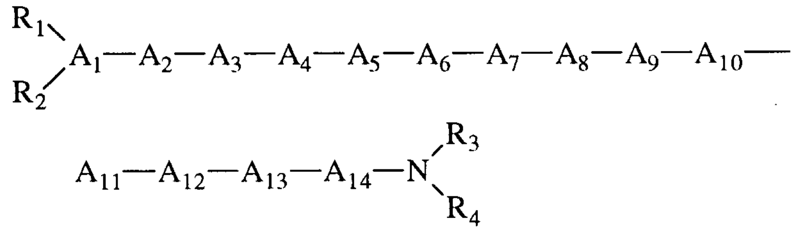

(I)

worin

- $A_1$ = pGlu, D oder L, oder es fehlt.
- $A_2$ = Gln, Asn, Gly, Ala, Leu, Ile, Nle, $\alpha$-Aminobuttersäure, Met, Val, Phe, p-X-Phe (X = F, Cl, Br, OH oder $CH_3$), Trp, $\beta$-Naphthylalanin oder es fehlt ;
- $A_3$ = Arg, D-Arg, Lys, D-Lys oder es fehlt ;
- $A_4$ = Gln, Asn, Gly, Ala, Leu, Ile, Nle, $\alpha$-Aminobuttersaüre, Met, Val, Phe, p-X-Phe (X = F, Cl, Br, OH oder $CH_3$), Trp, $\beta$-Naphthylalanin oder es fehlt ;
- $A_5$ = Gln, Asn, Gly, Ala, Leu, Ile, Nle, $\alpha$-Aminobuttersäure, Met, Val, Phe, D-Phe, p-X-Phe (X = F, Cl, Br, OH oder $CH_3$), Trp, $\beta$-Naphthylalanin, D-Ala oder es fehlt ;
- $A_5$ = Gln, Asn, Gly, Ala, D-Ala, N-Ac-D-Ala, Leu, Ile, Nle, $\alpha$-Aminobuttersaüre, Met, Val, Phe, p-X-Phe (X = F, Cl, Br, OH oder $CH_3$), Trp, p-Glu, $\beta$-Naphthylalanin oder es fehlt ;

$A_7$ = Gln, Asn, Gly, Ala, Leu, Ile, Nle, $\alpha$-Aminobuttersäure, Met, Val, Phe, D-Phe, p-X-Phe (X = F, Cl, Br, OH oder $CH_3$), Trp, Lys, His oder $\beta$-Naphthylalanin ;

$A_8$ = Trp, Met ;

$A_9$ = Gln, Asn, Gly, Ala, Leu, Ile, Nle, $\alpha$-Aminobuttersaüre, Met, Val, Phe, p-X-Phe (X = F, Cl, Br, OH oder $CH_3$), Trp oder $\beta$-Naphthylalanin, D oder L ;

$A_{10}$ = Gln, Asn, Gly, Ala, Leu, Ile, Nle, $\alpha$-Aminobuttersaüre, Met, Val, Phe, p-X-Phe (X = F, Cl, Br, OH oder $CH_3$), Trp, Thr oder $\beta$-Naphthylalanin ;

$A_{11}$ = Gly, Phe, D oder L ;

$A_{12}$ = His, Phe, D oder L oder p-X-Phe (X = F, Cl, Br, OH, $CH_3$), D oder L ;

$A_{13}$ = Gln, Asn, Gly, Ala, Leu, Ile, Nle, $\alpha$-Aminobuttersaüre, Met, Val, Phe, p-X-Phe (X = F, Cl, Br, OH oder $CH_3$), Trp oder $\beta$-Naphthylalanin ;

$A_{14}$ = Gln, Asn, Gly, Ala, Leu, Ile, Nle, $\alpha$-Aminobuttersäure, Met, Val, Phe, p-X-Phe (X = F, Cl, Br, OH oder $CH_3$), Trp oder $\beta$-Naphthylalanin ;

mit der Maßgabe, daß $R_1$, $R_2$, $R_3$ und $R_4$ jeweils unabhängig für H, $C_{1-12}$-Alkyl, $C_{7-10}$-Phenylalkyl, $COE_1$ (worin $E_1$ für $C_{1-20}$-Alkyl, $C_{3-20}$-Alkenyl, $C_{3-20}$-Alkinyl, Phenyl, Naphtyl oder $C_{7-10}$-Phenylalkyl steht) oder $COOE_2$ (worin $E_2$ für $C_{1-10}$-Alkyl oder $C_{7-10}$-Phenylalkyl steht) stehen, und $R_1$ und $R_2$ an die N-terminale Aminosaüre des genannten Peptids gebunden ist, die $A_1$, $A_2$, $A_3$, $A_4$, $A_8$, $A_8$ oder $A_7$ sein kann, und außerdem mit der Maßgabe, daß, wenn eines von $R_1$ oder $R_2$ für $COE_1$ oder $COOE_2$ steht, das andere H bedeuten muß, und wenn eines von $R_3$ oder $R_4$ $COE_1$ oder $COOE_2$ bedeutet, das andere H bedeuten muß, und außerdem mit der Maßgabe, daß, wenn $A_1$ = pGlu, $R_1$ H bedeuten und $R_2$ für den Teil von Glu stehen muß, der den Iminring in pGlu bildet, wobei für die Reste $A_7$, $A_8$, $A_9$, $A_{11}$, $A_{12}$ und $A_{13}$ jeweils unabhängig das Kohlenstoffatom, das an der Amidbindung zwischen diesem Rest und dein Stickstoffatom der $\alpha$-Aminogruppe des benachbarten Aminosäurerestes teilnimmt, ein Carbonylkohlenstoff sein kann oder zu einem Methylenkohlenstoff reduziert sein muß, oder ein pharmazeutisch verträgliches Salz davon.

**2.** Peptid nach Anspruch 1, worin $A_1$ bis $A_8$ einschließlich weggelassen werden, und das Kohlenstoffatom, das an der Amidbindung zwischen $Leu^{13}$ und $Leu^{14}$ teilnimmt, ein Methylenkohlenstoff ist, oder ein pharmazeutisch verträgliches Salz davon.

**3.** Peptid nach Anspruch 1, worin für jeden der genannten Reste $A_{11}$, $A_{12}$ und $A_{13}$ unabhängig das an der Amidbindung zwischen diesem Rest und dem Stickstoffatom der alpha-Aminogruppe des benachbarten Aminosäurerestes teilnehmende Kohlenstoffatom ein Carbonylkohlenstoff sein kann oder zu einem Methylenkohlenstoff reduziert sein kann, mit der Maßgabe, daß wenigstens ein solches Kohlenstoffatom zu einem Methylenkohlenstotf reduziert sein muß, oder ein pharmazeutisch verträgliches Salz davon.

**4.** Peptid der Aminosäureformel :

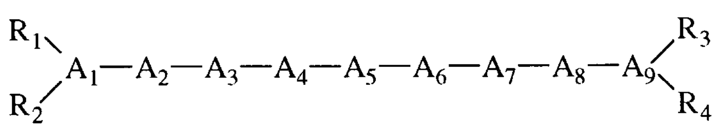

(II)

worin $A_1$ für $A_8$ nach Anspruch 1 steht, $A_2$ für $A_7$ nach Anspruch 1 steht, $A_3$ für $A_8$ nach Anspruch 1 steht, $A_4$ für $A_9$ nach Anspruch 1 steht, $A_8$ für $A_{10}$ nach Anspruch 1 steht, $A_8$ für $A_{11}$ nach Anspruch 1 steht, $A_7$ für $A_{12}$ nach Anspruch 1 steht, $A_8$ für $A_{13}$ nach Anspruch 1 steht und $A_9$ für $A_{14}$ nach Anspruch 1 steht, mit der Maßgabe, daß das Kohlenstoffatom, das an der Amidbindung zwischen dem $A_8$-Rest und dem Stickstoffatom der alpha-Aminogruppe des benachbarten Aminosäurerestes teilnimmt, zu einem Methylenkohlenstoff reduziert ist, oder ein pharmazeutisch verträgliches Salz davon.

**5.** Peptid nach Anspruch 1, worin für den Rest $A_{10}$ das Kohlenstoffatom, das an der Amidbindung zwischen diesem Rest und dein Stickstoffatom der alpha-Aminogruppe des benachbarten Aminosäurerestes teilnimmt, ein Carbonylkohlenstoff oder eine nichtpeptidische Bindung sein kann, mit der Maßgabe, daß die genannte nichtpeptidische Bindung der genannte Carbonylkohlenstoff ist, der zu

einem Methylenkohlenstoff reduziert worden ist, außerdem mit der Maßgabe, daß wenigstens ein solches Kohlenstoffatom zu einem Methylenkohlenstoff reduziert sein muß, oder ein pharmazeutisch verträgliches Salz davon.

**6.** Peptid mit der Aminosäureformel :
pGlu-Gln-Arg-Leu-Gly-Asn-Gln-Trp-Ala-Val-Gly-His-ψLeu [$CH_2NH$] Leu.

**7.** Peptid mit der Aminosäureformel nach Anspruch 1, das ein Analogon von natürlich auftretendem biologisch aktivem Säugetier-GRP darstellt oder Amphibien-Bombesin ist, das eine aktive Stelle aufweist, wobei die aktive Stelle die Positionen $A_9$, $A_{11}$, $A_{12}$, $A_{13}$ und $A_{14}$ und eine Bindungsstelle aufweist, die für die Bindung des genannten Bombesin an einen Rezeptor auf einer Ziel-zelle verantwortlich ist, wobei das genannte Analogon entweder (a) die genannte nichtpeptidische Bindung an Resten, außer innerhalb der genannten aktiven Stelle, aufweist, oder (b) wenigstens ein Statin- oder AHPPA-Rest anstelle von zwei natürlich auftretenen Aminosäuren der genannten aktiven Stelle aufweist und außerdem mit der Maßgabe, daß das Peptid Statin oder AHPPA enthalten kann, wenn alle Bindungen zwischen den Aminosäureresten Peptidbindungen sind, und außerdem mit der Maßgabe, daß, wenn ein Aminosäurerest Statin oder AHPPA darstellt, die Aminosäure rechts davon in der Formel weggelassen wird, so daß das genannte Analogon in der Lage ist, an den genannten Rezeptor zu binden und aufgrund des genannten Statin- oder AHPPA-Restes im Vergleich zu dem genannten natürlich vorkommenden Bombesin eine verstärkte biologische In vivo-Aktivität aufweist.

**8.** Peptid mit der Aminosäureformel :
pGlu-Gln-Arg-Leu-Gly-Asn-Gln-Trp-Ala-Val-Gly-His-Statin [$Sta^{13}$, Des $Met^{14}$] BN.

**9.** Verfahren zur Synthese eines Peptids der Aminosäureformel nach Anspruch 1, wobei das genannte Verfahren die Stufen umfaßt : (a) Herstellen von Benzhydrylamin-Polystyrolharz, (b) Neutralisieren des genannten Harzes und Kuppeln einer ausgewählten Aminosäure für die genannte Position $A_{14}$ an das genannte Harz, (c) Kuppeln einer gewählten Aminosäure für die genannte Position $A_{13}$ an das genannte $A_{14}$-Harz, (d) Derivatisierung der freien Aminogruppe des genannten $A_{13}$-$A_{14}$-Harzes, und (e) stufenweises Kuppeln an das genannte derivatisierte Aminosäureharz einer ausgewählten Aminosäure für jede der genannten Positionen $A_{12}$ bis $A_1$ durch Wiederholung der Stufen (d) und (e) für jede der genannten Aminosäurepositionen, wobei das Kohlenstoffatom, das an der Amidbindung zwischen einem der Reste $A_7$, $A_8$, $A_9$, $A_{11}$, $A_{12}$ und $A_{13}$ und dem Stickstoffatom der alpha-Aminogruppe des benachbarten Aminosäurerestes teilnimmt, zu einem Methylenkohlenstoff reduziert ist.

**10.** Verfahren nach Anspruch 9, bei dem irgendeine oder jede der genannten Positionen $A_8$ bis $A_1$ einschließlich weggelassen werden.

**11.** Verfahren zur Synthese eines Peptids der Aminosäureformel nach Anspruch 1, das ein Analogon von natürlich auftretendem biologisch aktivem Säuger-GRP oder von amphibischem Bombesin ist, das eine aktive Stelle, die die Positionen $A_9$, $A_{11}$, $A_{12}$, $A_{13}$ und $A_{14}$ einschließt und eine Bindungsstelle umfaßt, die für die Bindung an einen Rezeptor auf einer Zielzelle verantwortlich ist, wobei das genannte Verfahren eine der Stufen umfaßt :

- entweder (a) Herstellung des Benzhydrylamin-Polystyrolharzes, (b) Neutralisieren des genannten Harzes und Kuppeln einer ausgewählten Aminosäure für die genannte $A_{14}$-Aminosäureposition oder von alpha-t-Butoxycarbonyl-Statin an das genannte Harz, (c) Kuppeln einer gewählten Aminosäure für die genannte Position $A_{13}$ oder Statin für die genannte Position $A_{13}$ an das genannte $A_{14}$-Harz, (d) Derivatisierung der freien Aminogruppe des genannten $A_{13}$-$A_{14}$-Harzes oder des genannten Statin-Harzes, und (e) stufenweises Kuppeln an das genannte derivatisierte Aminosäure- oder Statinharz einer ausgewählten Aminosäure für jede der genannten Positionen $A_{12}$ bis einschließlich $A_1$ durch Wiederholung der Stufen (d) und (e) für jede der genannten Aminosäure-Positionen, mit der Maßgabe, daß, wenn das genannte Statin an das genannte Harz gekuppelt wird, das genannte Statinharz vor der Derivatisierung zur Freisetzung von Amino der Gruppen acetyliert wird, und mit der Maßgabe, daß wenigstens ein Statin- oder AHPPA-Rest die zwei natürlich vorkommenden Aminosäuren der genannten aktiven Stelle ersetzt,
- oder die Stufen des Verfahrens nach Anspruch 9, wenn die nichtpeptidische Bindung außerhalb der genannten aktiven Stelle liegt.

**12.** Peptid nach einem der Ansprüche 1 bis 8 zur Verwendung bei der Behandlung von Krebspatienten.

**13.** Verwendung eines Peptids nach einem der Ansprüche 1 bis 8 zur Herstellung eines Medikaments zur Verwendung bei der Behandlung von Krebspatienten.

**14.** Pharmazeutisches Präparat, umfassend ein Peptid nach einem der Ansprüche 1 bis 8 zusammen mit einem pharmazeutisch verträglichen Trägerstoff.

## Revendications

**1.** Un peptide ayant la formule d'aminoacides :

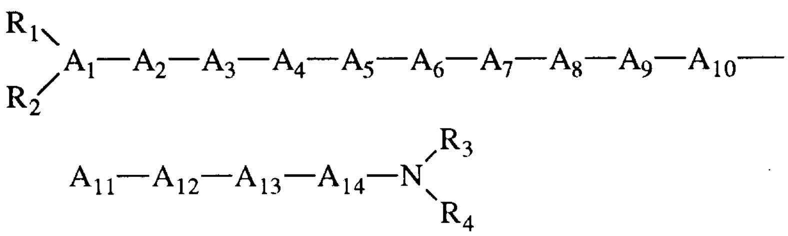

(I)

dans laquelle :

| | | |
|---|---|---|
| $A_1$ | = | pGlu, D ou L, ou est supprimé. |
| $A_2$ | = | Gln, Asn, Gly, Ala, Leu, Ile, Nle, acide $\alpha$-aminobutyrique, Met, Val, Phe, p-X-Phe (X = F, Cl, Br, OH ou $CH_3$), Trp, $\beta$-naphthylalanine ou est supprimé ; |
| $A_3$ | = | Arg, D-Arg, Lys, D-Lys ou est supprimé ; |
| $A_4$ | = | Gln, Asn, Gly, Ala, Leu, Ile, Nle, acide $\alpha$-aminobutyrique, Met, Val, Phe, p-X-Phe (X = F, Cl, Br, OH ou $CH_3$), Trp, $\beta$-naphthylalanine ou est supprimé ; |
| $A_5$ | = | Gln, Asn, Gly, Ala, Leu, Ile, Nle, acide $\alpha$-aminobutyrique, Met, Val, Phe, D-Phe, p-X-Phe (X = F, Cl, Br, OH ou $CH_3$), Trp, $\beta$-naphthylalanine, D-Ala ou est supprimé ; |
| $A_5$ | = | Gln, Asn, Gly, Ala, D-Ala, N-Ac-D-Ala, Leu, Ile, Nle, acide $\alpha$-aminobutyrique, Met, Val, Phe, p-X-Phe (X = F, Cl, Br, OH ou $CH_3$), Trp, p-Glu, $\beta$-naphthylalanine ou est supprimé ; |
| $A_7$ | = | Gln, Asn, Gly, Ala, Leu, Ile, Nle, acide $\alpha$-aminobutyrique, Met, Val, Phe, D-Phe, p-X-Phe (X = F, Cl, Br, OH ou $CH_3$), Trp, Lys, His, ou $\beta$-naphthylalanine ; |
| $A_5$ | = | Trp, Met ; |
| $A_9$ | = | Gln, Asn, Gly, Ala, Leu, Ile, Nle, acide $\alpha$-aminobutyrique, Met, Val, Phe, p-X-Phe (X = F, Cl, Br, OH ou $CH_3$), Trp, ou $\beta$-naphthylalanine, D ou L ; |
| $A_{10}$ | = | Gln, Asn, Gly, Ala, Leu, Ile, Nle, acide $\alpha$-aminobutyrique, Met, Val, Phe, p-X-Phe (X = F, Cl, Br, OH ou $CH_3$), Trp, Thr, ou $\beta$-naphthylalanine ; |
| $A_{11}$ | = | Gly, Phe, D ou L ; |
| $A_{12}$ | = | His, Phe, D ou L, ou p-X-Phe (X = F, Cl, Br, OH, $CH_3$), D ou L ; |
| $A_{13}$ | = | Gln, Asn, Gly, Ala, Leu, Ile, Nle, acide $\alpha$-aminobutyrique, Met, Val, Phe, p-X-Phe (X = F, Cl, Br, OH ou $CH_3$), Trp, ou $\beta$-naphthylalanine ; |
| $A_{14}$ | = | Gln, Asn, Gly, Ala, Leu, Ile, Nle, acide $\alpha$-aminobutyrique, Met, Val, Phe, p-X-Phe (X = F, Cl, Br, OH ou $CH_3$), Trp ou $\beta$-naphthylalanine ; |

étant entendu que

chacun de $R_1$, $R_2$, $R_3$ et $R_4$, indépendamment, est H, un alkyle en $C_{1-12}$, un phénylalkyle en $C_{7-10}$, $COE_1$ (où $E_1$ est un alkyle en $C_{1-20}$, un alkényle en $C_{3-20}$, un alkinyle en $C_{3-20}$, le groupe phényle, le groupe naphtyle, ou un phénylalkyle en $C_{7-10}$), ou $COOE_2$ (où $E_2$ est un alkyle en $C_{1-10}$ ou un phénylalkyle en $C_{7-10}$), et $R_1$ et $R_2$ sont liés à l'aminoacide N terminal dudit peptide, qui peut être $A_1$, $A_2$, $A_3$, $A_4$, $A_5$, $A_5$ ou $A_7$, et, en outre, étant entendu que lorsque $R_1$ ou $R_2$ est $COE_1$ ou $COOE_2$, l'autre doit être H, et lorsque $R_3$ ou $R_4$ est $COE_1$ ou $COOE_2$, l'autre doit être H, et étant en outre entendu que lorsque $A_1$ = pGlu, $R_1$ doit être H et $R_2$ doit être la partie de Glu qui forme le noyau imine dans pGlu ; et pour chacun des résidus $A_7$, $A_5$, $A_9$, $A_{11}$, $A_{12}$ et $A_{13}$, indépendamment, l'atome de carbone participant à la liaison amide entre ce résidu et l'atome d'azote du groupe alpha amino du résidu

d'aminoacides adjacent peut être un carbone de carbonyle ou peut être réduit à un carbone de méthylène, étant entendu qu'au moins un atome de carbone de ce genre doit être réduit à un carbone de méthylène ; ou un sel pharmaceutiquement acceptable de ce peptide.

**2.** Un peptide selon la revendication 1, caractérisé en ce que $A_1$ à $A_6$ inclus sont supprimés et que l'atome de carbone participant à la liaison amide entre $Leu^{13}$ et $Leu^{14}$ est un carbone de méthylène ; ou un sel pharmaceutiquement acceptable de ce peptide.

**3.** Un peptide selon la revendication 1, caractérisé en ce que pour chacun desdits résidus $A_{11}$, $A_{12}$ et $A_{13}$, indépendamment, l'atome de carbone participant à la liaison amide entre ce résidu et l'atome d'azote du groupe alpha amino du résidu d'aminoacides adjacent peut être un carbone de carbonyle ou peut être réduit à un carbone de méthylène, étant entendu qu'au moins un atome de carbone de ce genre doit être réduit à un carbone de méthylène ; ou un sel pharmaceutiquement acceptable de ce peptide.

**4.** Un peptide ayant la formule d'aminoacides

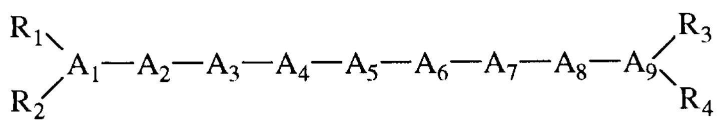

(II)

dans laquelle $A_1$ est $A_6$ de la revendication 1, $A_2$ est $A_7$ de la revendication 1, $A_3$ est $A_6$ de la revendication 1, $A_4$ est $A_9$ de la revendication 1, $A_6$ est $A_{10}$ de la revendication 1, $A_6$ est $A_{11}$ de la revendication 1, $A_7$ est $A_{12}$ de la revendication 1, $A_6$ est $A_{13}$ de la revendication 1 et $A_9$ est $A_{14}$ de la revendication 1 ; étant entendu que l'atome de carbone participant à la liaison amide entre le résidu $A_6$ et l'atome d'azote du groupe alpha amino du résidu d'aminoacides adjacent est réduit à un carbone de méthylène ; ou un sel phalmaceutiquement acceptable de ce peptide.

**5.** Un peptide selon la revendication 1, caractérisé en ce que pour le résidu $A_{10}$, l'atome de carbone participant à la liaison amide entre ce résidu et l'atome d'azote du groupe alpha amino du résidu d'aminoacides adjacent peut être un atome de carbonyle ou peut être une liaison non-peptide, étant entendu que ladite liaison non-peptide est ledit carbone de carbonyle qui a été réduit à un carbone de méthylène, étant en outre entendu qu'au moins un atome de carbone de ce genre doit être réduit à un carbone de méthylène ; ou un sel pharmaceutiquement acceptable de ce peptide.

**6.** Un peptide ayant la formule d'aminoacides
pGlu-Gln-Arg-Leu-Gly-Asn-Gln-Trp-Ala-Val-Gly-His-$\psi$Leu [$CH_2NH$] Leu.

**7.** Un peptide ayant la formule d'aminoacides de la revendication 1, qui est un analogue de PLG de mammifères ou de la bombésine d'amphibiens biologiquement actifs, naturels, comportant un site actif, ledit site actif comprenant les positions $A_9$, $A_{11}$, $A_{12}$, $A_{13}$ et $A_{14}$ et un site de liaison responsable de la fixation de ladite bombésine à un récepteur sur une cellule-cible, ledit analogue comportant soit (a) ladite liaison non-peptide à des résidus autres que ceux situés à l'intérieur dudit site actif, soit (b) au moins un résidu statine ou AHPPA à la place de deux aminoacides naturels dudit site actif, et étant en outre entendu que le peptide peut contenir de la statine ou de l'AHPPA lorsque toutes les liaisons entre les résidus d'aminoacides sont des liaisons peptides, et étant en outre entendu que lorsqu'un résidu d'aminoacides est la statine ou l'AHPPA, l'aminoacide à sa droite dans la formule est supprimé, de telle sorte que ledit analogue est capable de se fixer audit récepteur et, en raison dudit résidu de statine ou d'AHPPA, de présenter une activité biologique in vivo accrue en comparaison de ladite bombésine naturelle.

**8.** Un peptide ayant la formule d'aminoacides
pGlu-Gln-Arg-Leu-Gly-Asn-Gln-Trp-Ala-Val-Gly-His-Statine [$Sta^{13}$, Des $Met^{14}$] BN.

**9.** Une méthode de synthèse d'un peptide ayant la formule d'aminoacides définie dans la revendication 1 ; ladite méthode comprenant les phases suivantes : (a) préparation d'une résine benzhydrylamine-polystyrène, (b) neutralisation de ladite résine et couplage d'un aminoacide choisi pour ladite position

$A_{14}$ à ladite résine, (c) couplage d'un aminoacide choisi pour ladite position $A_{13}$ à ladite résine $A_{14}$, (d) dérivatisation du groupe amino libre de ladite résine $A_{13}$-$A_{14}$ et (e) couplage successivement à ladite résine d'aminoacides dérivatisée d'un aminoacide choisi pour chacune desdites positions $A_{12}$ à $A_1$ en répétant les phases (d) et (e) pour chacune desdites positions d'aminoacides, l'atome de carbone participant à la liaison amide entre l'un quelconque des résidus $A_7$, $A_8$, $A_9$, $A_{11}$, $A_{12}$ et $A_{13}$ et l'atome d'azote du groupe alpha amino du résidu d'aminoacides adjacent étant réduit à un carbone de méthylène.

**10.** La méthode de la revendication 9, caractérisée en ce que l'une quelconque desdites ou toutes lesdites positions $A_8$ à $A_1$ incluse sont supprimées.

**11.** Une méthode de synthèse d'un peptide ayant la formule d'aminoacides de la revendication 1, qui est un analogue de PLG de mammifères ou de bombésine d'amphibiens, biologiquement actifs, naturels, qui comprend un site actif incluant les positions $A_9$, $A_{11}$, $A_{12}$, $A_{13}$ et $A_{14}$ et un site de liaison responsable de la fixation à un récepteur sur une cellule-cible, ladite méthode comprenant :

- soit les phases (a) préparation d'une résine benzhydrylamine-polystyrène, (b) neutralisation de ladite résine et couplage d'un aminoacide choisi pour ladite position d'aminoacide $A_{14}$ ou de l'alpha-t-butoxycarbonyl statine à ladite résine, (c) couplage d'un aminoacide choisi pour ladite position $A_{13}$ ou de ladite statine pour ladite position $A_{13}$ à ladite résine $A_{14}$, (d) dérivatisation du groupe amino libre de ladite résine $A_{13}$-$A_{14}$ ou de ladite résine-statine, et (e) couplage successivement audit aminoacide dérivatisé ou à ladite statine-résine d'un aminoacide choisi pour chacune desdites positions $A_{12}$ à $A_1$ incluse par répétition des phases (d) et (e) pour chacune desdites positions d'aminoacides ; étant entendu que lorsque ladite statine est couplée à ladite résine, ladite statine-résine est acétylée avant dérivatisation du groupe amino libre ; et étant entendu qu'au moins un résidu de statine ou d'AHPPA remplace deux aminoacides naturels dudit site actif ;
- soit les phases de la méthode de la revendication 9, lorsque la liaison non-peptide se trouve à l'extérieur dudit site actif.

**12.** Un peptide selon l'une quelconque des revendications 1 à 8, destiné à être utilisé dans le traitement du cancer chez les patients.

**13.** Utilisation d'un peptide selon l'une quelconque des revendications 1 à 8 pour la fabrication d'un médicament destiné à être utilisé dans le traitement du cancer chez les patients.

**14.** Une composition pharmaceutique comprenant un peptide selon l'une quelconque des revendications 1 à 8, conjointement avec un support pharmaceutiquement acceptable.